# EUROPÄISCHE PATENTANMELDUNG

(11) **EP 3 569 187 A1**
(43) Veröffentlichungstag der Anmeldung: **20.11.2019**
(21) Anmeldenummer: 18173249.6
(22) Anmeldetag: 18.05.2018
(51) Int. Cl.: A61C 13/00, A61C 9/00, A61C 19/05, A61F 5/56

(54) **VERFAHREN ZUR HERSTELLUNG VON AUFBISSSCHIENEN**

(71) Anmelder: Weber, Joachim, 67071 Ludwigshafen (DE); Image Instruments GmbH, 09125 Chemnitz (DE)
(72) Erfinder: Weber, Joachim, 67071 Ludwigshafen (DE); Meyer, Elisabeth, 67071 Ludwigshafen (DE)
(74) Vertreter: Reitstötter Kinzebach

(57) **Zusammenfassung**

Die vorliegende Erfindung betrifft ein Verfahren zur Herstellung von Aufbissschienen und Folge-Aufbissschienen zur Verwendung in der Behandlung eines Patienten mit wenigstens einer Erkrankung, die von einer funktionellen Störung des Kauorgans ausgeht, insbesondere Kraniomandibulärer Dysfunktionen, sowie Aufbissschienen und Folge-Aufbissschienen, die nach diesem Verfahren erhältlich sind.

## Beschreibung

Die vorliegende Erfindung betrifft ein Verfahren zur Herstellung von Aufbissschienen und Folge-Aufbissschienen zur Verwendung in der Behandlung eines Patienten mit wenigstens einer Erkrankung, die von einer funktionellen Störung des Kauorgans ausgeht, insbesondere Kraniomandibulärer Dysfunktionen, sowie Aufbissschienen und Folge-Aufbissschienen, die nach diesem Verfahren erhältlich sind.

### Hintergrund der Erfindung

Patienten mit Erkrankungen des Kauorgans stellen einen wesentlichen Bestandteil der zahnärztlichen Behandlungen, insbesondere kieferorthopädischen Behandlungen, dar. Eine im zahnärztlichen Alltag häufig anzutreffende Erkrankung, die von einer funktionale Störung des Kauorgans ausgeht, ist die sogenannte Kraniomandibuläre Dysfunktion (Cranium = Schädel; Mandibula = Unterkiefer) oder auch Myoarthropathie, die im Folgenden als CMD bezeichnet wird. Die CMD ist gekennzeichnet durch muskuloskelettale Pathologien des kraniomandibulären Systems eines Patienten. Ein Kiefergelenk besteht im Wesentlichen aus einem Gelenkkopf (Condylus mit Caput mandibulae) des Unterkiefers, der beweglich in einer Gelenkgrube (Fossa articularis) des Schläfenbeins angeordnet ist. Der Unterkiefer ist dabei durch ein komplexes System aus Knorpel, Muskeln und Bändern, insbesondere der bilaminären Zone am Schädel aufgehängt, wodurch sich die Lage des Kiefergelenkes und die Okklusion, d.h. der Schlussbiss der Zähne des Ober- und Unterkiefers, gegenseitig in Statik und Dynamik beeinflussen. Eine ideale Okklusion besteht, wenn die Zähne in einer physiologischen Position zueinander stehen und der Kiefer dabei seine physiologische Lage behalten kann.

Die Kaumuskellage ist wesentlich an der Kopfhaltung in Statik und Dynamik beteiligt. Die Kopfhaltung hat wiederum maßgeblichen Einfluss auf die gesamte Körperhaltung. Dadurch entstehen häufig Wechselwirkungen der Kaumuskellage mit dem Halteapparat im Nackenbereich während dieser wiederum mit der gesamten Körperstatik interagiert.

Die häufig auftretende Kraniomandibuläre Dysfunktion ist eine pathologische und damit nicht physiologische Lage des Gelenkkopfes in der Gelenkgrube und eine damit zusammenhängende Über- oder Fehlbelastung der gesamten beteiligten anatomischen Strukturen, wie Bänder, Knorpel, Muskulatur, Zähne und der Kiefergelenke. Dies führt in der Regel zu schmerzhaften Verspannungen der Kaumuskulatur bis hin zu dauerhafter Schädigung von Bändern, Sehnen und Knorpel sowie zur Destruktion beteiligter knöcherner Strukturen. Zu den Symptomen der CMD gehören Kopf-, Ohren- oder Gesichtsschmerzen sowie das nächtliche Pressen und Knirschen der Zähne. Daneben können weitere Beschwerden, die nicht direkt mit dem Kausystem in Verbindung stehen, wie Tinnitus, Sehstörungen, Schlafstörungen oder Gleichgewichtsstörungen oder Probleme im Bereich der Wirbelsäule sowie des gesamten Bewegungsapparates, z.B. der Hüfte, der Beine, der Knie, der Füße und des Schulterbereichs, auftreten.

Bei der Behandlung von CMD werden häufig Aufbissschienen, nachfolgend auch als Schiene oder Zahnschiene bezeichnet, eingesetzt. Ziel des Einsatzes solcher Aufbissschienen ist es, den Patienten von einer pathologischen Okklusion und/oder Kaubewegung abzuhalten bzw. ihm diese abzugewöhnen. In der Folge soll die physiologische Zentrik der Gelenkköpfe in den Gelenkgruben des Kiefergelenks wiederhergestellt werden. Damit soll die Über- und Fehlbelastung der Zähne und Kiefergelenke zumindest teilweise beseitigt werden, was in der Regel zu einer verbesserten Positionierung der Kiefergelenke und einer Relaxierung der Kiefermuskulatur führt. Dadurch können Entzündungen abklingen, sich Knochenstrukturen remodellieren und sich der Bewegungsapparat rekalibrieren.

Von der Gestaltung her unterscheidet man grundsätzlich Bissschienen mit nichtadjustierter, d.h. nicht an das Kauprofil des Patienten angepasste Kaufläche, und Bissschienen mit adjustierten Kauflächen. Die Schienen können Teile der Zahnreihe (einzelne Zähne) bedecken oder sich über alle Zähne eines Kiefers erstrecken.

Im Stand der Technik sind verschiedene Aufbissschienen zur Behandlung der Kraniomandibulären Dysfunktion sowie entsprechende Verfahren zur Herstellung solcher Aufbissschienen bekannt.

Die Gebrauchsmusterschrift DE202004020196 U1 beschreibt eine Oberkiefer-Aufbissschiene mit einem Aufnahmebereich für Oberkieferzähne sowie mit einer im eingesetzten Zustand der Aufbissschiene zu den Unterkieferzähnen gerichteten Unterseite, wobei die Unterseite der Aufbissschiene im Frontzahnbereich als ein Plateau (Frontzahn-Schienen-Plateau) ausgebildet ist, welches das vollständige Schließen der Kiefer verhindert, d.h. die Kiefer in einem leicht geöffneten Zustand belässt. Der Aufnahmebereich für die Oberkieferzähne ist derart ausgestaltet, dass das Plateau im eingesetzten Zustand der Aufbissschiene mit der Okklusionsebene einen Winkel im Bereich von 0° bis 10° bzw. in einer weiteren Ausgestaltung einen Winkel im Bereich von 5° bis 30° bildet. Die Schienen weisen keine Eckzahnführung und Seitenzahnkontakte auf. Durch die leichte Öffnung der Kiefergelenke und die fehlenden Eckzahnführung und Seitenzahnkontakte soll die physiologische Zentrik der Kiefergelenke (CR-Position) wiederhergestellt und eine neuromuskuläre Relaxation der Kiefergelenke und Kiefermuskulatur erreicht werden.

Die Gebrauchsmusterschrift DE202009003999 U1 beschreibt eine Aufbissschiene für den Ober- oder Unterkiefer, bestehend aus einer Basisplatte mit passgenauen Zahnauflageelementen, wobei die Zahnauflageelemente eine erste Fläche aufweisen, die okklusal an die Zähne angepasst ist, und einer zweite Fläche aufweisen, die vestibulär zur zweiten Ebene angeordnet ist und eine Seitenzahnführung gewährleisten, und wobei die Zahnauflageelemente insgesamt eine kraniale konkave Kalottenform aufweisen. Die Aufbissschienen können zudem Ausgleichselemente aufweisen welche die Anpassung der Okklusion bei der Behandlung eines Patienten mit CMD über den Therapieverlauf ermöglichen.

Die DE102013112032A1 beschreibt ein Verfahren zum Konstruieren einer adjustierten Aufbissschiene zur Verwendung bei der Behandlung eines Patienten mit mindestens einer Kraniomandibulären Dysfunktion aus einer bestehenden Aufbissschiene. Dieses Verfahren umfasst die computergestützten Schritte: Erfassen von Daten zum eindeutigen Definieren einer Form der bestehenden Aufbissschiene, einer aktuellen Morphologie der Zähne des zweiten Kiefers und einer Lage des zweiten Kiefers, des Kontaktbereiches der bestehenden Aufbissschiene und von Kiefergelenken des Patienten relativ zueinander; Simulieren einer Okklusion eines kraniomandibulären Systems des Patienten, das die in den Patienten eingesetzte bestehende Aufbissschiene umfasst; Identifizieren von Störkontakten zwischen dem Kontaktbereich der bestehenden Aufbissschiene und den Zähnen des zweiten Kiefers; und Konstruieren der adjustierten Aufbissschiene aus den erfassten Daten und aus der Simulation gewonnenen Daten, sodass die identifizierten Störkontakte eliminiert sind. Des Weiteren wird vorgeschlagen, die vorgenannten Schritte zu wiederholen, um eine Serie von Aufbissschienen zur iterativen Annäherung an die physiologische Lage der Kiefergelenke zu konstruieren.

Die im Stand der Technik bekannten Verfahren zur Herstellung von Aufbissschienen zur Behandlung von CMD, gehen in der Regel von einer idealen Bissposition aus, d.h. einer Bissposition mit zahnärztlich definierter Okklusion, Stellung des Unterkiefers bzw. der Gelenkköpfe sowie zahnärztlich definierter muskulärer Haltung. Unter dieser "ideale Bissposition" wird in aller Regel die physiologische Bissposition verstanden, die auch als zentrische Kondylenposition bzw. "centric relation position" (CR-Position) oder als "stable condyle position" (SCP) bezeichnet wird. Die ideale (physiologische) Bissposition kann sich jedoch über den Zeitraum der Behandlung der Funktionsstörung des Kauorgans ändern. Zudem kann die ideale Bissposition je nach Kopf- und Körperhaltungen bzw. je nach Körperposition des Patienten (z.B. Stehen, Liegen, Sitzen) und je nachdem ob der Patient eine ruhende Position einnimmt oder in Bewegung ist (Statik versus Dynamik) unterschiedlich sein, d.h. es können in Abhängigkeit der individuellen Einsatzbereiche der Aufbissschienen durch den Patienten mehrere ideale Bisspositionen existieren.

In diesem Zusammenhang wäre es wünschenswert ein Verfahren zur Herstellung von Aufbissschienen zur Behandlung von CMD zur Verfügung zu haben, mit dem sich auch mehrere Aufbissschienen, die jeweils optimal an die verschiedenen relevanten Körperhaltungen des Patienten bzw. Einsatzzwecke angepasst sind, herstellen lassen (beispielsweise eine Schiene für den Tag und eine für die Nacht). Daneben werden bei den im Stand der Technik bekannten Verfahren, die von einer idealen (physiologischen) Bissposition ausgehen, Untersuchungsbefunde aus von der Kieferorthopädie verschiedenen medizinischen Bereichen gar nicht oder nur unzureichend berücksichtigt, obwohl diese einen erheblichen Einfluss auf die erfolgreiche Behandlung einer Erkrankung, die von einer funktionellen Störung des Kauorgans ausgeht, insbesondere von Kraniomandibulären Dysfunktionen, haben können.

Es ist daher die Aufgabe der vorliegenden Erfindung, ein Verfahren zur Herstellung von Aufbissschienen und Folge-Aufbissschienen zur Verwendung in der Behandlung eines Patienten mit wenigstens einer Erkrankung, die von einer funktionellen Störung des Kauorgans ausgeht, bereitzustellen, welches gegenüber den im Stand der Technik bekannten Verfahren eine schnellere, effektivere und individuellere, d.h. insbesondere auf die interdisziplinären Befunde und auf verschiedenen relevante Körperhaltungen des Patienten bzw. den individuellen Einsatzzwecken abgestimmte, Behandlung der Kraniomandibulären Dysfunktionen ermöglicht.

Diese Aufgabe wird erfindungsgemäß durch ein Verfahren zur Herstellung von Aufbissschienen und Folge-Aufbissschienen zur Verwendung in der Behandlung eines Patienten mit wenigstens einer Erkrankung, die von einer funktionellen Störung des Kauorgans ausgeht, insbesondere Kraniomandibulärer Dysfunktionen, gelöst, wie in den Patentansprüchen und im Folgenden detailliert beschrieben wird.

Das erfindungsgemäße Verfahren zeichnet sich insbesondere dadurch aus, dass aufgrund der Berücksichtigung mehrerer Bisspositionen Aufbissschiene(n), welche die Beschwerden des Patienten weitgehend eliminieren oder zumindest reduzieren, schneller und effektiver aufgefunden werden können. Die Berücksichtigung verschiedener patientenspezifischer Kopf- und Körperhaltungen bzw. Körperpositionen in Statik als auch in Dynamik, ermöglicht zudem eine gezielt auf die individuellen Bedürfnisse des Patienten abgestimmte Behandlung der CMD. Durch das Einbeziehen von Befunden aus von der Zahnheilkunde und Kieferorthopädie verschiedenen medizinischen Bereichen, kann das Ergebnis der Behandlung noch weiter verbessert werden. Nicht zuletzt können mit dem erfindungsgemäßen Verfahren die Aufbissschienen kostengünstig und reproduzierbar hergestellt werden. Mit dem erfindungsgemäßen Verfahren ist es auch möglich zu jedem Zeitpunkt der Therapie zu früheren Aufbissschienen zurückzukehren, falls mit einer allfälligen Folge-Aufbissschiene nicht die gewünschte Verbesserung erzielt werden kann. Es kann zudem gezielt und schnell sowohl auf gewünschte Veränderungen (z.B. kompensatorische Prozesse) als auch auf ungewünschte Veränderungen (z.B. dekompensatorische Prozesse) während der Behandlung der CMD reagiert werden.

### Zusammenfassung der Erfindung

Die vorliegende Erfindung betrifft ein Verfahren zur Herstellung von Aufbissschienen und Folge-Aufbissschienen zur Verwendung in der Behandlung eines Patienten mit wenigstens einer Erkrankung, die von einer funktionellen Störung des Kauorgans ausgeht, insbesondere Kraniomandibulärer Dysfunktionen, enthaltend die folgenden Schritte:
a) Erfassen von Daten zur Lage der Kiefer, der Kiefergelenke, der Bewegungsbahnen des Unterkiefers und der aktuellen Morphologie der Zähne des Patienten (Ausgangs-Zustand),
b) Bestimmung von zwei oder mehr als zwei verschiedenen Bisspositionen des Patienten,
c) computergestützte Zusammenführung der in Schritt a) erfassten Daten (Ausgangs-Zustand) und der in Schritt b) bestimmten Bisspositionen (Soll-Zustände T₀) zur Berechnung jeweils eines temporären Modells einer Aufbissschiene für zwei oder mehr als zwei der in Schritt b) bestimmten Bisspositionen,
d) Erzeugung jeweils einer Aufbissschiene für zwei oder mehr als zwei der in Schritt b) bestimmten Bisspositionen, basierend auf den in Schritt c) berechneten temporären Modellen,
e) Tragen der Aufbissschienen durch den Patienten,
f) Auswahl einer oder mehrerer der in Schritt e) getragenen Aufbissschienen oder Verwerfung aller in Schritt e) getragenen Aufbissschienen und
g) Erzeugung von Folge-Aufbissschienen.

Ferner betrifft die vorliegende Erfindung eine Aufbissschiene und Folge-Aufbissschienen, zur Verwendung in der Behandlung eines Patienten mit wenigstens einer Erkrankung, die von einer funktionellen Störung des Kauorgans ausgeht, insbesondere Kraniomandibulärer Dysfunktionen, erhältlich durch ein Verfahren wie zuvor und im Folgenden beschreiben.

### Detaillierte Beschreibung der Erfindung

Die hier verwendeten Begriffe "Aufbissschiene" sowie "Folge-Aufbissschiene" bezeichnet ein Mittel zum Aufbeißen für den Oberkiefer und/oder Unterkiefer, der wenigstens einen Aufnahmebereich zur Aufnahme der Zähne des Oberkiefers und/oder Unterkiefers und/oder wenigstens einen Kontaktbereich zum Kontaktieren der Zähne des Oberkiefers und/oder des Unterkiefers aufweist. Eine "Folge-Aufbissschiene" kann dabei als Ersatz für die erste Schiene tatsächlich im Sinne eines totalen Ersatzes der ersten Schiene nachfolgen, beispielsweise wenn die erste Schiene aus therapeutischen Gründen abgeändert wurde oder ganz verworfen werden musste. Der Begriff "Folge-Aufbissschiene" wird jedoch auch verwendet für in der Produktion zeitlich nachfolgender Schienen, die jedoch gleichzeitig zum therapeutischen Einsatz kommen, beispielsweise in Kombinationen wie Tag-/Nacht-Schienen, Sport-/Arbeits-Schienen, Hart-/Weich-Schienen und dergleichen.

Der Aufnahmebereich der Aufbissschienen und der Folge-Aufbissschienen kann nicht profiliert, d.h. nicht an die jeweilige Zahnmorphologie (Kauprofil) des Patienten angepasst sein, geringfügig profiliert, d.h. geringfügig oder grob an die jeweilige Zahnmorphologie des Patienten angepasst sein, oder auch stark profiliert, d.h. vollständig an die jeweilige Zahnmorphologie des Patienten angepasst sein. Der Kontaktbereich wiederum kann entweder nicht profiliert, d.h. nicht an die jeweilige Zahnmorphologie (Kauprofil) des Patienten angepasst sein, sodass eine vollständige Bewegungsfreiheit der Kiefer zueinander in der maximalen Kontaktposition bestehen bleibt, oder geringfügig profiliert, d.h. geringfügig oder grob an die jeweilige Zahnmorphologie des Patienten angepasst sein, sodass die Bewegungsfreiheit der Kiefer zueinander in der End-Bissstellung eingeschränkt wird. Umfasst werden z.B. sowohl Okklusionsschienen, wie Äquilibrierungsschienen, Repositionsschienen, Vertikalisationsschienen, Dekompressionsschienen und Protrusionsschienen; sowie Reflexschienen, z. B. mit beidseitigem Aufbiss oder anteriorem Aufbiss.

In diesem Zusammenhang verwendeten Ausdrücke "geringfügig profiliert" bzw. "geringfügig oder grob an die Zahnmorphologie des Patienten angepasst" bedeuten, dass die Profilierung keine formschlüssige Negativabbildung (Abdruck) der Kontaktbereiche der Zähne mit der Aufbissschiene darstellt, sondern dass die Profilierung aus zur Oberfläche der Aufbissschiene hin erweiterten Vertiefungen bzw. Gruben und/oder Rampen im Kontaktbereich der Zähne besteht (okklusale Impressionen). Anders ausgedrückt handelt es sich hierbei um geglättete Abdrücke der Kontaktbereiche der Zähne. Die Glättung der Kontaktbereiche kann mehr oder weniger stark ausgeprägt sein, je nachdem wie groß die gewünschte Bewegungsfreiheit der Kiefer in der End-Bissstellung sein soll.

Sinngemäß bedeuten die Ausdrücke "stark profiliert" bzw. "vollständig an die Zahnmorphologie des Patienten angepasst", dass die Profilierung eine im Wesentlichen formschlüssige Negativabbildung (Abdruck) des Kontaktbereichs der Zähne des Patienten darstellt, welches die Bewegungsfreiheit der Kiefer in der End-Bissstellung stark einschränkt oder sogar vollständig unterbindet.

Der wenigstens eine Aufnahmebereich zur Aufnahme der Zähne des Oberkiefers und/oder Unterkiefers weist bevorzugt eine geringe bis starke Profilierung auf. Besonders bevorzugt ist der wenigstens eine Aufnahmebereich stark profiliert, d.h. vollständig an die jeweilige Zahnmorphologie (Kauprofil) des Patienten angepasst.

Der Aufnahmebereich kann zusätzlich Mittel zum Führen der Zähne aufweisen. Bevorzugt weist der Aufnahmebereich wenigstens ein Mittel zum Führen der Zähne auf. Bei dem Mittel zum Führen der Zähne kann es sich beispielsweise um eine Frontzahnführung, Eckzahnführung, Gruppenführung (z.B. bei Malokklusionen), balancierte Okklusionen (z.B. bei Zahnersatz) oder Seitenzahnführung sowie um Kombinationen davon handeln. Bevorzugt ist das Mittel zum Führen der Zähne ausgewählt unter einer Frontzahnführung, Eckzahnführung und einer Front-Eckzahn-Führung, insbesondere einer Front-Eckzahn-Führung.

Bevorzugt weist der Kontaktbereich eine geringfügige Profilierung auf, d.h. der Kontaktbereich ist geringfügig oder grob an die jeweilige Zahnmorphologie des Patienten angepasst. Damit sollen die Kiefer in der End-Bissstellung in eine gewünschte Position dirigiert werden.

Bevorzugt weisen die durch das erfindungsgemäße Verfahren erhältliche Aufbissschienen und Folge-Aufbissschienen einen Aufnahmebereich zum Aufnehmen von Zähnen eines ersten Kiefers des Patienten und einen Kontaktbereich zum Kontaktieren von Zähnen eines dem ersten Kiefer gegenüberliegend angeordneten zweiten Kiefers des Patienten. Der Aufnahmebereich dieser Aufbissschienen und Folge-Aufbissschienen ist so profiliert, dass ein ausreichend sicherer Halt der Schienen auch bei Belastung (z.B. Beißen, Sprechen, Bruxismus) gewährleistet ist. Gegebenenfalls kann der Halt der Schienen mit üblichen Haltemitteln (z.B. Knopfanker) verbessert werden. Der Kontaktbereich ist vorzugsweise geringfügig oder grob an die jeweilige Zahnmorphologie des Patienten angepasst. Weiterhin bevorzugt weisen diese Aufbissschienen und Folge-Aufbissschienen Mittel zum Führen der Zähne, wie oben definiert, z.B. eine Front-Eckzahn-Führung auf.

Die Stärke der Aufbissschiene bzw. der Folge-Aufbissschiene, d.h. der Abstand des Aufnahmebereichs oder Kontaktbereichs für den ersten Kiefer zum Aufnahmebereich oder Kontaktbereich für den dem ersten Kiefer gegenüberliegend angeordneten zweiten Kiefer, kann stark variieren und orientiert sich an dem Ausmaß der therapeutisch gewünschten Bisserhöhung durch die Kiefer- bzw. Mundöffnung im Bedarf mit oder ohne Lageveränderung durch z.B. Protrusion und/oder Laterotrusion.

Der hier verwendeten Begriff "End-Bissstellung", auch Okklusion in Zentrik genannt, bezeichnet den Endpunkt der Kieferschlussbewegung mit maximalem Vielpunktkontakt der Zähne mit den gegenüberliegenden Zähnen oder dem Material, auf das die Zähne beißen. Idealerweise sollten in der End-Bissstellung beide Gelenkköpfe zentral in den jeweiligen Gelenkgruben sitzen. Dabei sollten das linke und rechte Kiefergelenk sich jeweils zentrisch in ihren Gelenkgruben befinden, die Kaumuskulatur auf beiden Seiten jeweils physiologische Spannung und Länge aufweisen und die Zahnreihen im Seitenzahngebiet rechts und links gleichmäßige Vielpunktkontakte zeigen. Diese Parameter werden unbewusst, z. B. beim Schlucken vom Gehirn überprüft. Falls Fehlbelastungen vorliegen, setzt ein unwillkürlicher Kompensationsmechanismus über neurologische Reflexbahnen ein, sodass die Person den Unterkiefer verschiebt bis sie wieder beiderseits Vielpunktkontakte an den Zähnen spürt. Allerdings geht dabei die Symmetrie und die beidseitig gleichmäßige Spannung der Muskulatur verloren, was z. B. zu Bruxismus und Kraniomandibulären Dysfunktionen führt.

Der hier verwendete Begriff "Kieferstellung" bezeichnet eine Stellung des Unterkiefers und Oberkiefers zueinander, wobei sowohl physiologische als auch pathologische Kieferstellungen umfasst werden. Pathologische Kieferstellungen können primär auftreten oder sekundär durch andere Störungen bedingt sein, wie z.B. eine Fehlstellung der Zähne.

Der hier verwendeten Begriffe "Ausgangs-Zustand" und "Ist-Zustand" bezeichnen die gemessene bzw. aktuelle anatomische Lagen der Kiefer, der Kiefergelenke sowie die vorliegende Zahnmorphologie. Der hier verwendete Begriff "Soll-Zustand" bezeichnet eine erwünschte anatomische Lage der Kiefer, der Kiefergelenke sowie der vorliegenden Zahnmorphologie, die z. B. dem physiologischen Zustand entspricht. Der Soll-Zustand kann beispielsweise anhand von Tabellenwerten (z.B. cephalometrische Vermessungen, Baugut-Tabellen, gnatologische Normwerte, usw.) aus den anatomischen Gegebenheiten des Patienten berechnet oder auch, wie nachfolgend ausgeführt, klinisch und empirisch bestimmt werden.

Um die hohen Datenmengen, die unter anderem durch den Einbezug verschiedener Untersuchungsbefunde (ggf. aus verschiedene Fachgebieten) und dem iterativen Charakter des Verfahrens anfallen, und die damit einhergehende Komplexität des vorliegenden Verfahrens handhaben zu können, werden die einzelnen Verfahrensschritte zur Herstellung der Aufbissschienen und Folge-Aufbissschienen wenn möglich computergestützt durchgeführt, wobei hierzu geeignete Computerprogramme (Software) zum Einsatz kommen.

### Schritt a):

In einem ersten Schritt des erfindungsgemäßen Verfahrens werden Daten zur Lage der Kiefer, der Kiefergelenke, der Bewegungsbahnen des Unterkiefers und der aktuellen Morphologie der Zähne des Patienten erfasst.

Hierzu eignen sich prinzipiell alle im Stand der Technik bekannten Verfahren zur Erfassung anatomischer Daten, die mittels Computer-Programmen eingelesen, bildlich bzw. graphisch dargestellt und bearbeitet werden können. Das Erfassen der Daten erfolgt vorzugsweise mittels dreidimensionaler Bildgebungsverfahren. In diesem Zusammenhang bezeichnet der Begriff "dreidimensionales Bildgebungsverfahren" jegliche bildliche Wiedergabe eines realen Objektes in drei bis vier Dimensionen, z. B. durch x-, y-, z- und Zeit-Koordinaten. Dreidimensionale Bildgebungsverfahren umfassen beispielsweise dreidimensionales Röntgen, Kernspintomographie oder Magnetresonanztomographie (MRT), dreidimensionaler Computertomographie (CT) bzw. digitale Volumentomographie und/oder Oberflächenrasterung durch Auflichtscannen. Die Bilddaten können sowohl analog als auch digital wiedergegeben werden, z.B. durch individuelle oder kommerziell erhältliche Computer-Programme, wie z.B. DiCom (OFFIS e.V., Oldenburg, Deutschland). Der Begriff "dreidimensionales Bildgebungsverfahren" umfasst auch ein Übereinanderlagern und Bearbeiten von Bilddaten, z. B. mittels Computer-Programmen, wie coDiagnostiX@ (Chemnitz, Deutschland), Onyx-Ceph (Image Instruments GmbH), 3shape (3Shape, Kopenhagen, Dänemark) oder implant3D (med3D GmbH, Heidelberg, Deutschland). Aus den Bilddaten lassen sich Ist-Werte als Koordinaten, Längen-, Volumen und Winkelmaße abmessen.

Daneben stehen dem Fachmann spezifische Verfahren zur Beschreibung der Lagebeziehung der anatomischen Strukturen in Statik und Dynamik (z.B. Erfassung der Bewegungsbahnen des Unterkiefers durch die Okklusionsanalyse und achsiographische Untersuchungsverfahren) zur Verfügung, aus denen sich für die Kiefergelenke und die Bewegungsbahnen des Unterkiefers relevante biomechanische Parameter, wie beispielsweise die Scharnierachse, Zentrik, Kondylenbahnneigung, Bennett-Winkel oder Bennett-Bewegung bestimmen lassen. Für das erfindungsgemäße Verfahren eignen sich prinzipiell alle im Stand der Technik bekannten Verfahren zur Erfassung der Bewegungsbahnen des Unterkiefers, welche Daten zu den Bewegungsbahnen in digitaler Form liefern, die mittels Computer-Programmen eingelesen, bildlich oder graphisch dargestellt und gegebenenfalls digital bearbeitet werden können. Kommerziell erhältliche Systeme zur Erfassung der Bewegungsbahnen des Unterkiefers sind beispielsweise das JMAnalyser+ System von Zebris, das Freecorder®BlueFox-System der DDI Group oder das DMD-System von Ignident.

Die in Schritt a) erhobenen Daten dienen als Grundlage für die Konstruktion der Aufbissschiene und Folge-Aufbissschiene nach dem erfindungsgemäßen Verfahren. Hieraus lassen sich die für die Therapie wesentliche Parameter, die bei der Konstruktion der Schienen berücksichtigt werden müssen, berechnen, wie beispielsweise die Materialstärke, Materialart, Oberflächenstruktur in Anpassung an die Kieferposition in Statik und Dynamik und dergleichen. Für Folge-Schienen kann ausgehend von der Ausgangs-Schiene (Initialschiene) die genaue metrisch gewünschte Veränderung berechnet werden.

Falls der Patient bereits eine bestehende Aufbissschiene besitzt, werden in Schritt a) des erfindungsgemäßen Verfahrens zusätzlich die Form der bestehenden Aufbissschiene und der Kontaktbereich der Zähne mit der Aufbissschiene mittels der oben beschriebenen Verfahren erfasst. Diese Daten werden bei der Konstruktion der neuen Aufbissschienen und Folge-Aufbissschienen berücksichtigt.

### Schritt b):

In einem nächsten Schritt des erfindungsgemäßen Verfahrens werden zwei oder mehr als zwei verschiedene Bisspositionen des Patienten bestimmt.

In diesem Zusammenhang bezieht sich der Begriff "Bissposition" auf eine, für die Therapie der von einer funktionellen Störung des Kauorgans ausgehenden Erkrankung (insbesondere Kraniomandibulärer Dysfunktionen) relevante bzw. potentiell/vermutlich relevante Lage des Ober- und Unterkiefers relativ zueinander. Die Bissposition stellt somit einen Soll-Zustand, d.h. eine erwünschte anatomische Lage der Kiefer und der Kiefergelenke dar. Der hier verwendete Begriff "Bissposition" ist von dem Begriff "ideale Bissposition", wie oben definiert, zu unterscheiden. Die in Schritt b) bestimmten Bisspositionen können die ideale (physiologische) Bissposition enthalten, umfassen aber auch weitere Bisspositionen, die sich in der Regel von der idealen Bissposition unterscheiden, beispielsweise aufgrund verschiedener Körperpositionen und Körperpositionen in Statik und Dynamik und/oder durch den Einbezug von Untersuchungsbefunden aus von der Kieferorthopädie verschiedenen medizinischen Bereichen.

Die Bestimmung dieser für die Therapie der von einer funktionellen Störung des Kauorgans ausgehenden Erkrankung relevanten Bisspositionen kann auf verschiedene Weise erfolgen.

In einer ersten Ausführungsform des erfindungsgemäßen Verfahrens erfolgt die Bestimmung der von einer funktionellen Störung des Kauorgans ausgehenden Erkrankung relevanten Bisspositionen in Schritt b) unter Einsatz der zuvor unter Schritt a) beschriebenen Verfahren, und über weiter empirische und klinische Verfahren.

Werden für die Bestimmung der relevanten Bisspositionen die zuvor unter Schritt a) beschriebenen Verfahren eingesetzt, erfolgt die Bestimmung der Bissposition bei den für den Patienten jeweils maßgeblichen Kopf- und Körperhaltungen bzw. bei verschiedenen Körperpositionen in Statik als auch Dynamik (wie z.B. Stehen, Sitzen, Liegen aber auch Laufen, Gehen, usw.). Beispielsweise können unter Zuhilfenahme der Magnetresonsanztomographie (MRT) verschiedene Bisspositionen bestimmt werden, die aus anatomischer bzw. biomechanischer Sicht positive Auswirkungen auf die Beschwerden des Patienten haben sollten (z.B. Entlastung entzündlicher Bereiche, genügend große Kiefergelenkspaltbreite, usw.).

Zusätzlich zu den in Schritt a) verwendeten Verfahren zur Bestimmung der relevanten Bisspositionen können weiteren empirischen und klinischen Verfahren eingesetzt werden. Hiernach erfolgt die Bestimmung der Bisspositionen beispielsweise durch das Einsetzen von Abstandshaltern oder Teilschienen zwischen dem Ober- und Unterkiefer, Auflageteilschienen, die gegebenenfalls kurzzeitig über (eine) etwaige bestehende Initialschiene(n) aufgestülpt werden können, oder mit Hilfe von (Biss)-Registraten, welche die Kiefer temporär in eine bestimmte Position fixieren, bei der die Beschwerden des Patienten zumindest teilweise reduziert sind. Auch diese empirische Bestimmung kann bei einer bestimmten Kopf- und Körperhaltung, z.B. aufrechtes gerades Stehen, oder bei unterschiedlichen Kopf- und Körperhaltungen bzw. bei verschiedenen Körperpositionen in Statik und Dynamik (wie z.B. Stehen, Sitzen, Liegen aber auch Laufen, Gehen, usw.) erfolgen. Bevorzugt erfolgt die empirische und klinische Bestimmung der Bisspositionen bei einer bestimmten Kopf- und Körperhaltung, z.B. aufrechtes gerades Stehen, oder bei unterschiedlichen Kopf- und Körperhaltungen bzw. bei verschiedenen Körperpositionen in Statik und Dynamik (wie z.B. Stehen, Sitzen, Liegen aber auch Laufen, Gehen, usw.).

Diese empirische und klinische Bestimmung der Bisspositionen eignet sich vor allem bei Beschwerden bzw. Symptomen, die nach einer zumindest teilweisen Behebung der Ursache schnell, z.B. innerhalb weniger Minuten bis wenigen Stunden, nachlassen oder weitgehend verschwinden können und objektiv messbar sind. Bei diesen Beschwerden bzw. Symptomen kann es sich beispielsweise um die folgenden Beschwerden bzw. Symptome handeln:
- Gleichgewichtsstörungen, wie beispielsweise Schwankschwindel, verursacht durch Wechselwirkung der Kaumuskelgruppe mit der Nackenmuskulatur und Belastung der Halswirbelsäule, die sich objektiv durch Messen und Analyse der Körperschwankungen z.B. mit Posturographiegeräten (z.B. dem Posturographiegerät Tetrax der Firma BeamMed Ltd., Israel), bestimmen lassen;
- Tinnitus oder Blutflussgeräusche bei Patienten, die unter nächtlichem Zähnepressen oder Knirschen (Bruxismus) leiden; objektiv bestimmbar durch Messung der Hirnströme;
- Sehstörungen, die sich objektiv über entsprechende Sehtests bestimmen lassen;
- Muskelverspannungen, wie Verspannungen der Kaumuskelgruppe, Nacken- oder Rückenmuskulatur, die sich objektiv durch Messungen des Muskeltonus bestimmen lassen.

In einer bevorzugten Ausführungsform des erfindungsgemäßen Verfahrens erfolgt die Bestimmung der Bisspositionen in Schritt b) bei unterschiedlichen Kopf- und Körperhaltungen bzw. bei verschiedenen Körperpositionen in Statik und Dynamik.

Des Weiteren können Untersuchungsbefunde aus von der Kieferorthopädie verschiedenen medizinischen Bereichen in die Bestimmung der für die Therapie der funktionellen Erkrankung des Kauorgans relevanten Bisspositionen mit einbezogen werden. Hierdurch lassen sich die durch die CMD hervorgerufenen Beschwerden des Patienten in der Regel noch gezielter und effektiver behandeln.

In eine weiteren bevorzugten Ausführungsform des erfindungsgemäßen Verfahrens erfolgt die Bestimmung der Bisspositionen in Schritt b) daher unter Einbezug eines oder mehrerer Untersuchungsbefunde aus von der Kieferorthopädie verschiedenen medizinischen Bereichen.

Diese Untersuchungsbefunde können je nach Relevanz unterschiedlich stark gewichtet werden, d.h. mehr oder weniger stark bei der Bestimmung der für die Therapie der funktionellen Erkrankung des Kauorgans relevanten Bisspositionen berücksichtigt werden.

Diese Untersuchungsbefunde können prinzipiell aus allen der Kieferorthopädie angrenzenden medizinischen Fachbereichen, wie z.B. HNO (insbesondere die Gleichgewichtsorgane) oder der Augenheilkunde und/oder aus allen medizinischen Bereichen, die sich mit der Kopf- bzw. Körperhaltung in Statik und Dynamik befassen, wie die Osteopathie, Physiotherapie, Orthopädie, Sportmedizin, Logopädie, Manualtherapie, Chirotherapie, und dergleichen, stammen.

Bei den Untersuchungsbefunden aus von der Kieferorthopädie verschiedenen medizinischen Bereichen, welche in die Bestimmung der für die Therapie der funktionellen Erkrankung des Kauorgans relevanten Bisspositionen mit einfließen können, kann es sich beispielsweise um die nachfolgend aufgeführten Untersuchungsbefunde handeln.

Beispielsweise können osteopathische Gesamtkörperbefunde in die Bestimmung der für die Therapie der funktionellen Erkrankung des Kauorgans relevanten Bisspositionen mit einbezogen werden. Hierzu werden die aus osteopathischer Sicht günstige Gesamtkörperhaltung erfasst und dabei die Kieferhaltung kontrolliert. So können z.B. in besonders entspannten Kopf- und Körperpositionen und/oder in einer blockadelösenden Gesamtkörperhaltung eine oder mehrere Bisspositionen erhoben werden. Die so aus osteopathischer Sicht ermittelte optimale Kieferstellung für die Gesamtkörperhaltung wird registriert und in die Bestimmung der für die Therapie der funktionellen Erkrankung des Kauorgans relevanten Bisspositionen mit einbezogen.

Des Weiteren können orthopädische Gesamtkörperbefunde in die Bestimmung der für die Therapie der funktionellen Erkrankung des Kauorgans relevanten Bisspositionen mit einbezogen werden. Hierzu werden die orthopädischen Gesamtkörperbefunde, beispielsweise mittels Laufbandanalyse, Analyse der Körperhaltung wie beispielsweise mit der Diers-Gerätetechnik oder mittels Röntgen-, CT-, MRT-Analysen, erfasst und dabei die Kieferhaltung kontrolliert. Dabei werden für die Gesamtkörperhaltung relevante Erkrankungen, wie Gelenk- und Haltungsveränderungen im Rahmen rheumatischer Erkrankungen, Fußgewölbefehlstände, Fehlständen der Wirbelsäule und dergleichen, sowie geplante und/oder bereits bestehende Therapien bzw. Eingriffe, wie z.B. die Konstruktion von Schuheinlagen, Planung der Körperhaltung beim Einfügen von künstlichen Gelenken oder Skoliosetherapie, mit einbezogen. Die so aus orthopädischer Sicht ermittelte optimale Kieferstellung für die Gesamtkörperhaltung wird registriert und in die Bestimmung der für die Therapie der funktionellen Erkrankung des Kauorgans relevanten Bisspositionen mit einbezogen.

Des Weiteren können HNO Gesamtkörperbefunde in die Bestimmung der für die Therapie der funktionellen Erkrankung des Kauorgans relevanten Bisspositionen mit einbezogen werden. Die Wechselwirkung zwischen der funktionellen Erkrankung des Kauorgans und dem HNO medizinischen Bereich ergibt sich hier häufig bei den bereits oben erwähnten Beschwerden bzw. Symptomen, wie Tinnitus, Blutflussgeräuschen oder Gleichgewichtsstörungen. Hierzu werden die HNO Gesamtkörperbefunde erfasst und dabei die Kieferhaltung kontrolliert. Die so aus HNO-medizinischer Sicht ermittelte optimale Kieferstellung wird registriert und in die Bestimmung der für die Therapie der funktionellen Erkrankung des Kauorgans relevanten Bisspositionen mit einbezogen.

Daneben können Gesamtkörperbefunde aus weiteren medizinischen Bereichen, die sich mit der Körperhaltung in Statik und Dynamik beschäftigen, wie beispielsweise Befunde aus der Sportmedizin, Physiotherapie, Logopädie, Manualtherapie, Chirotherapie und dergleichen in die Bestimmung der für die Therapie der funktionellen Erkrankung des Kauorgans relevanten Bisspositionen mit einbezogen werden.

Die Registrierung der Kieferstellung kann über herkömmliche zahnmedizinische Verfahren zur Erstellung von Abdrücken mittels Registraten, wie Wachse, Silikonbisse (z.B. aus Vinylpolysiloxan), schnellhärtenden Polymerschäumen und dergleichen, erfolgen. Daneben können auch dreidimensionale Bildgebungsverfahren, wie oben definiert, insbesondere Oberflächenrasterung der Mundhöhle durch Auflichtscannen, zur Registrierung der Kieferstellung eingesetzt werden.

Bevorzugt sind die Untersuchungsbefunde aus von der Kieferorthopädie verschiedenen medizinischen Bereichen, die in die Bestimmung der Bisspositionen in Schritt b) mit einbezogen werden ausgewählt unter
- aus osteopathischer Sicht optimale Kopf- und Körperhaltung,
- aus orthopädischer Sicht optimale Körperhaltung,
- aus HNO Sicht optimale Kopfhaltung und Kieferstellung,
- aus augenärztlicher Sicht optimale Kopf- und Körperhaltung.

Die oben genannten Bestimmungsmethoden der relevanten Bisspositionen können beliebig miteinander kombiniert werden. Beispielsweise kann die aus biomechanischer Sicht ideale Bissposition, die mittels bildgebender Verfahren bestimmt wurden, mit empirisch und klinisch bestimmt Bisspositionen aus einem von der Kieferorthopädie verschiedenen medizinischen Bereichs kombiniert werden.

In einer zweiten Ausführungsform des erfindungsgemäßen Verfahrens werden die für die Therapie der funktionellen Erkrankung des Kauorgans relevanten Bisspositionen aus den in Schritt a) erfassten Daten, wie beispielsweise Schädel-CT, DVT, MRT, berechnet. Hierbei wird nicht zwingend davon ausgegangen, dass eine ganz bestimmte ideale Bissposition, die üblicherweise der biomechanisch idealen Bissposition entspricht, existiert, sondern vielmehr ausgehend von verschiedenen medizinischen Annahmen (z.B. über die Höhe der Aufbissschiene oder der Form der Impressionen), die beispielsweise auf der Erfahrung des behandelnden Arztes basieren, mehrere mögliche für die Therapie relevante Bisspositionen ausschließlich berechnet. Bevorzugt werden auch in dieser zweiten Ausführungsform des erfindungsgemäßen Verfahrens unterschiedliche Kopf- und Körperhaltungen bzw. verschiedenen Körperpositionen berücksichtigt.

Die so in Schritt b) des erfindungsgemäßen bestimmten Bisspositionen stellen eine Biss-Bibliothek des Patienten dar. Jede einzelne Bissposition aus dieser Biss-Bibliothek kann dabei als Ausgangspunkt für die Herstellung einer oder mehrerer Aufbissschienen für einen bestimmten Einsatzzweck bzw. zur Herstellung von sogenannten "Begleitschienen" für weitere Einsatzzwecke verwendet werden.

Der in dem vorliegenden Verfahren verfolgte zentrale Ansatz, bei der Behandlung von Kraniomandibulären Dysfunktionen von mehreren patientenspezifischen Bisspositionen auszugehen, ermöglicht eine ganzheitliche und hochgradig patientenspezifische Behandlung der CMD. Dieser Ansatz erweist sich insbesondere in den Fällen als besonders vorteilhaft, in denen

- in der Dynamik eine andere Form erforderlich ist als in der Statik (insbesondere Sport);
- bei Positionsveränderungen (Stehen, Sitzen, Liegen) eine andere Form erforderlich ist (beispielsweise individualisierte Schnarch- oder Apnoeschienen)
- aus ästhetischen Gründen andere Bauformen erforderlich sind (z.B. Minischiene ohne Frontzahnerhöhung für Tags);
- aus verschiedenen Fachbereichen divergierende Hinweise zur optimalen Position vorliegen (probatorische Testschienen);
- aus verschiedenen Fachbereiche divergierende Therapieaufgaben vorliegen (z.B. Trainingsschienen in unterschiedlicher Form für Logo-, Physio-, Ergotherapie und Dergleichen).

Insgesamt können alle Faktoren auch bei der Herstellung von kieferorthopädischen Korrekturschienen (Alignern) berücksichtigt werden.

### Schritt c):

In einem nächsten Schritt des erfindungsgemäßen Verfahrens erfolgt die computergestützte Zusammenführung der in Schritt a) erfassten Daten (Ausgangs-Zustand) und der in Schritt b) bestimmten Bisspositionen (Soll-Zustände T₀) zur Berechnung jeweils eines temporären Modells einer Aufbissschiene für zwei oder mehr als zwei der in Schritt b) bestimmten Bisspositionen

Hierzu werden die in Schritt a) erfassten Daten und die in Schritt b) bestimmten Bisspositionen mittels einer geeigneten Computersoftware bildlich dargestellt. Hierzu eignen sich prinzipiell alle Programme, mit denen dreidimensionaler (Bild)-Daten verarbeitet, d.h. dargestellt, bearbeitet und berechnet werden können, wie beispielsweise Programme für Computer-Aided Design bzw. Computer-Aided Manufacturing (CAD/CAM-Programme). Bei der bildgebenden Darstellung werden sowohl Oberflächen- als auch Volumendaten des Schädels inkl. der Zähne des Patienten sowie Bewegungsbahnen bzw. Bewegungswolken des Unterkiefers (Ausgangs-Zustand) sowie die in Schritt b) bestimmten Bisspositionen (Soll-Zustände T₀) in einer Gesamtdarstellung zusammengeführt, wie z. B. durch die Überlagerung von Bilddaten. Diese Überlagerung kann manuell durch den behandelnden Arzt oder unter Zuhilfenahme einer geeigneten Software (virtuelles Matching) erfolgen. Fehlstellungen der Zähne, der Kiefer und des Kiefergelenks werden durch die bildgebende Darstellung sichtbar und lassen sich über anatomische Fixpunkte, die als Referenzpunkte dienen, quantifizieren.

Anschließend werden die in Schritt b) bestimmten Bisspositionen (Soll-Zustände T₀) mit dem Ausgangs-Zustand verglichen, um ein Maß einer Abweichung des Ist-Zustandes von den jeweiligen gewünschten Soll-Zuständen und damit eine Maß einer therapeutischen Repositionierung in die jeweiligen gewünschten Soll-Zustände zu bestimmen.

Hierbei können auch zwei oder mehrere Bisspositionen, die auf verschiedenen Untersuchungsbefunden, z.B. auf verschiedenen Untersuchungsbefunden aus von der Kieferorthopädie verschiedenen medizinischen Bereichen, basieren, zu einer Bissposition zusammengeführt werden. Hierbei können die zusammengeführten Bisspositionen gleich (Mittelung), oder, je nach Relevanz, unterschiedlich stark gewichtet werden.

Die aus den Analysen der Soll-Zuständen und dem Ist-Zustand gewonnenen Maße der Abweichungen werden auf einen virtuellen Artikulator lagerichtig übertragen, anhand dessen temporäre Modelle einer Aufbissschiene für jede der gewünschten Bisspositionen (Soll-Zustände) berechnet werden, die den Kiefer in die gewünschten Lagen repositionieren. Ein virtueller Artikulator ist mit einer Software gleichzusetzen, die 3-dimensionale Daten verarbeitet.

Falls gewünscht, ist es selbstverständlich auch möglich, die gewünschten Bisspositionen auf einen physikalischen Artikulator zu übertragen. Hierzu werden die temporären Modelle, die Negativabbildungen der gewünschten Bisspositionen darstellen, beispielsweise mittels 3D-Druckverfahren gedruckt und an dem physikalischen Artikulator lagerichtig angebracht.

### Schritt d):

Anschließend wird jeweils eine Aufbissschiene für zwei oder mehr als zwei der in Schritt b) bestimmten Bisspositionen, basierend auf den in Schritt c) berechneten temporären Modellen, erzeugt.

Die Ausgabe der Aufbissschienen und Folge-Aufbissschienen erfolgt in vorteilhafter Weise mit 3D formgebenden Geräten, wie z.B. 3D-Druckern und 3D-Fräseinheiten. Bei der Verwendung von 3D-Druckern und 3D-Fräseinheiten wird üblicherweise so vorgegangen, dass zunächst ein Positiv-Modell der Zahnmorphologie mit der temporären Ideallagen der Kiefer und der Kiefergelenke gedruckt wird, aus dem dann die Schienen, beispielsweise durch Tiefziehen eines geeigneten Polymermaterials, hergestellt werden können.

### Schritt e):

Die erzeugten Aufbissschienen werden anschließend durch den Patienten getragen.

Jede Schiene wird üblicherweise nacheinander oder bei verschiedenen Einsatzzwecken auch parallel, beispielsweise eine Aufbissschiene für den Tag und eine andere Aufbissschiene für die Nacht, über einen Zeitraum von mehreren Tagen, beispielsweise über 3, 4, 5, 6, oder 7 Tage, bis einigen Wochen, beispielsweise über 2 oder 3 Wochen getragen. Bevorzugt werden die Schienen über einen Zeitraum von 1 bis 2 Wochen getragen.

### Schritt f):

Anschließend wird/werden eine oder mehrere der in Schritt e) getragenen Aufbissschienen ausgewählt, oder gegebenenfalls alle in Schritt e) getragenen Aufbissschienen verworfen.

Die Auswahl der Aufbissschiene(n) erfolgt in der Regel anhand des objektiven patientenspezifischen Nutzens und/oder anhand des subjektiven Empfindens des Patienten.

Die Beurteilung des objektiven patientenspezifischen Nutzens erfolgt dabei üblicherweise durch den Kieferorthopäden über eine manuelle Befundkontrolle unter Verwendung empirischer und klinischer Untersuchungsmethoden, wie oben beschrieben. Zusätzlich oder alternativ dazu können mittels dreidimensionaler bildgebender Verfahren Daten über die aktuellen Lage der Kiefer, der Kiefergelenke sowie der Zahnmorphologie für die jeweiligen getragenen Aufbissschienen erhoben und in die Beurteilung des objektiven patientenspezifischen Nutzens mit einbezogen werden. Zum Beispiel können die anatomischen Auswirkungen, die durch das Tragen einer bestimmten Aufbissschiene hervorgerufen werden, wie beispielsweise die Entlastung entzündlicher Bereiche oder die Kiefergelenkspaltbreite, mittels MRT gezielt überprüft werden.

Falls Untersuchungsbefunde aus einem von der Kieferorthopädie verschiedenen medizinischen Bereichs in die Bestimmung der Bisspositionen in Schritt b) mit einbezogen wurden, kann die Beurteilung des objektiven patientenspezifischen Nutzens zusammen mit einem Arzt aus diesem medizinischen Bereich erfolgen.

Bei dieser Beurteilung ist in der Regel jedoch das subjektive Empfinden des Patienten maßgeblich.

In einer bevorzugten Ausführungsform des erfindungsgemäßen Verfahrens umfasst die Auswahl der einen oder mehreren Aufbissschienen die folgenden Schritte:
f.1) gegebenenfalls die Erfassung von Daten zur Bestimmung der aktuellen Lage der Kiefer, der Kiefergelenke sowie der Zahnmorphologie für die getragenen Aufbissschienen und/oder die Erfassung weiterer empirischer und klinischer Daten aus von der Kieferorthopädie verschiedenen medizinischen Bereichen für die getragenen Aufbissschienen (aktueller Ist-Zustand),
f.2) Auswahl einer oder mehrerer Aufbissschienen anhand des aus den gegebenenfalls in Schritt f.1) erfassten Daten bestimmten objektiven patientenspezifischen Nutzens und/oder anhand des subjektiven Empfindens des Patienten.

Falls der aktuellen Ist-Zustandes gemäß Schritt f.1) bestimmt wird, erfolgt dieser üblicherweise mit eingesetzter Schiene.

In einer besonders bevorzugten Ausführungsform des erfindungsgemäßen Verfahrens erfolgt die Auswahl der Aufbissschiene(n) überwiegend anhand des subjektiven Empfindens des Patienten, wobei gegebenenfalls kieferorthopädischer Gesichtspunkte mitberücksichtigt werden.

### Schritt g):

Bei der Therapie der CMD wird der Patient an ein neues muskuläres Bewegungsmuster gewöhnt, die zu einer therapeutisch günstigen Lage der Kiefer und der Kiefergelenke führt. Diese Gewöhnung ist ein kontinuierlicher Vorgang und erfolgt über einen längeren Zeitraum, üblicherweise über mehrere Monate, in dem der Körper im Sinne eines Regelkreises reagiert. Daher müssen die nach der initialen Tragephase ausgewählten Aufbissschienen in der Regel angepasst und/oder ersetzt werden.

Hierzu werden in einem weiteren Schritt g) des erfindungsgemäßen Verfahrens aus den in Schritt f) ausgewählten Aufbissschienen (nachfolgend auch als Initialschiene(n) bezeichnet) Folge-Aufbissschienen erzeugt.

Dies kann je nach vorliegender Situation und Umsetzbarkeit in unterschiedlicher Weise erfolgen. Beispielsweise können, für den Fall, dass wenigstens eine Aufbissschiene in Schritt f) für gut befunden und ausgewählt wurde, leichte Abwandlungen dieser Initialschiene(n) als Folge-Schienen erzeugt werden. Diese leichten Abwandlungen können die Form der Initialschiene(n) (z.B. Dicke der Schiene, Verbesserung der Impressionen/Profilierung oder der auf der Schiene angebrachten Mittel zur Zahnführung, und dergleichen) und/oder das Material aus dem die Schiene(n) aufgebaut ist/sind (z.B. zur Modifizierung der Bissbeständigkeit oder der Plastizität der Schiene) betreffen. Des Weiteren können, für den Fall, dass in Schritt f) mehrere Schienen ausgewählt wurden, auch aus zwei oder mehr als zwei dieser Initialschienen eine oder mehrere gemittelte Schienen als Folge-Schiene(n) erstellt werden. Die erwähnten Folge-Schienen können dann alternativ zu der/den bestehenden Initialschiene(n) getragen werden oder die Initialschiene(n) auch ersetzen. Letzteres gilt insbesondere für den Fall einer iterativen Annäherung an eine gewünschte Bissposition durch den Einsatz mehrerer Zwischenstufen (Zwischenschienen), wie unten ausgeführt. Für den Fall, dass in Schritt f) alle getragenen Schienen verworfen werden, müssen üblicherweise neue Bisspositionen bestimmt werden, die den individuellen Bedürfnissen des Patienten besser Rechnung tragen. Basierend auf diesen neuen Bisspositionen werden dann Folge-Schienen erzeugt, die als neue Initialschienen von dem Patienten getragen werden.

Zur Erzeugung der Folge-Aufbissschienen werden in einem ersten Schritt üblicherweise Daten zur Bestimmung der aktuellen Lage der Kiefer, der Kiefergelenke sowie der Zahnmorphologie für die in Schritt f) ausgewählten Aufbissschienen und/oder weitere empirische und klinische Daten aus von der Kieferorthopädie verschiedenen medizinischen Bereichen für die in Schritt f) ausgewählten Aufbissschienen (aktueller Ist-Zustand) erfasst, falls dies nicht bereits in Schritt f) erfolgt ist. Anhand dieser Daten kann die aktuelle Lage der Kiefer und der Kiefergelenke bei eingesetzter Schiene mit der jeweils gewünschten Bissposition verglichen werden.

In einem nächsten Schritt werden zur jeweiligen in Schritt f) ausgewählten Aufbissschiene (Initialschiene) neue Bisspositionen bestimmt und/oder berechnet. In der Regel unterscheiden sich diese Bisspositionen von der ursprünglichen Bissposition nur geringfügig und dienen der weiteren Annäherung an die gewünschte Bissposition oder zur Erzeugung alternativer Aufbissschienen.

Zur Bestimmung der neuen Bisspositionen können prinzipiell alle unter Schritt b) beschriebenen empirischen und klinischen Verfahren eingesetzt werden, wobei vorzugsweise Abstandshalter und Auflageteilschienen, die über die bestehende Aufbissschiene aufgestülpt bzw. an diese fixiert werden können, eingesetzt werden.

Daneben oder alternativ können die neuen Bisspositionen aus der bestehenden Aufbissschiene berechnet werden. Hierzu können die im vorangegangenen Schritt erhobenen oder gegebenenfalls bereits in Schritt f) erhobenen Daten zur Bestimmung der aktuellen Lage der Kiefer, der Kiefergelenke sowie der Zahnmorphologie und/oder die weiterer empirischer und klinischer Daten aus von der Kieferorthopädie verschiedenen medizinischen Bereichen mit einbezogen werden.

Die erhobenen Daten zur bestehenden Aufbissschiene und der neu bestimmten Bisspositionen werden zusammengeführt und jeweils ein temporäres Modell einer Aufbissschiene für die zuvor bestimmten und/oder berechneten neuen Bisspositionen berechnet. Aus den temporären Modellen werden dann die Folge-Aufbissschienen erzeugt. Die Berechnung der Modelle bzw. die Erzeugung der Folge-Aufbissschienen erfolgt analog zu den oben beschriebenen Schritten c) und d).

Falls in Schritt f) eine oder mehrere Aufbissschienen ausgewählt wurden (Initialschienen), umfasst die Erzeugung der Folge-Aufbissschienen in Schritt g) bevorzugt die folgenden Schritte:
g.1a) die Erfassung von Daten zur Bestimmung der aktuellen Lage der Kiefer, der Kiefergelenke sowie der Zahnmorphologie für die in Schritt f) ausgewählten Aufbissschienen und/oder die Erfassung weiterer empirischer und klinischer Daten aus von der Kieferorthopädie verschiedenen medizinischen Bereichen für die in Schritt f) ausgewählten Aufbissschienen (aktueller Ist-Zustand), falls dies nicht bereits in Schritt f) erfolgt ist,
g.2a) Bestimmung und/oder Berechnung neuer Bisspositionen basierend auf den in Schritt f) ausgewählten Aufbissschienen,
g.3a) computergestützte Zusammenführung der in Schritt g.1a) erfassten Daten (aktueller Ist-Zustand) und der in Schritt g.2a) bestimmten und/oder berechneten Bisspositionen zur Berechnung jeweils eines temporären Modells einer Aufbissschiene für zwei oder mehr als zwei der in Schritt g.2a) bestimmten und/oder berechneten Bisspositionen, und
g.4a) Erzeugung jeweils einer Folge-Aufbissschiene für zwei oder mehr als zwei der in Schritt g.2a) bestimmten und/oder berechneten neuen Bisspositionen, basierend auf den in Schritt g.3a) berechneten temporären Modellen.

Wie zuvor erwähnt, können je nach vorliegender Situation die Folge-Aufbissschienen alternativ zu den Initialschienen getragen werden oder die Folge-Aufbissschienen die Initialschienen ersetzen.

Falls keine der in Schritt e) getragenen Aufbissschienen für gut befunden und daher in Schritt f) verworfen wurden, müssen neue Bisspositionen bestimmt werden, die den Anforderungen des Patienten besser Rechnung tragen.

In diesem Fall umfasst die Erzeugung von Folge-Aufbissschienen vorzugsweise die folgenden Schritte:
g.1b) Bestimmung von zwei oder mehr als zwei neuen Bisspositionen des Patienten,
g.2b) computergestützte Zusammenführung der in Schritt a) erfassten Daten (aktueller Ist-Zustand) und der in Schritt g.1b) bestimmten neuen Bisspositionen zur Berechnung jeweils eines temporären Modells einer Aufbissschiene für zwei oder mehr als zwei der in Schritt g.1b) bestimmten Bisspositionen und
g.3b) Erzeugung jeweils einer Folge-Aufbissschiene für zwei oder mehr als zwei der in Schritt g.1b) bestimmten Bisspositionen, basierend auf den in Schritt g.2b) berechneten temporären Modellen.

Die Bestimmung dieser neuen Bisspositionen in Schritt g.1b) erfolgt wie oben unter Schritt b) beschrieben. Die Schritte g.2b) und g.3b) erfolgen analog zu den oben beschriebenen Schritten c) und d).

### Iteratives Vorgehen:

Wie zuvor erwähnt erfolgt die Therapie der CMD häufig über einen längeren Zeitraum in dem der Körper im Sinne eines Regelkreises reagiert, was eine mehrfache Anpassung der Aufbissschiene erforderlich machen kann, bis sämtliche funktionellen Symptome verschwunden sind und eine stabile physiologische Lage der Gelenkköpfe in den Gelenkgruben erreicht ist. Des Weiteren kann es im Rahmen von CMD Behandlungen neben Fortschritten immer auch Rückfälle (Rezidive) geben, die eine Anpassung der Aufbissschienen, d.h. die Herstellung von Folge-Aufbissschienen, oder sogar eine Rückkehr zu einer initialen Aufbissschiene erforderlich machen. Daneben kann auch ein Wechsel zu einer auf einer alternativen Bissposition basierenden Aufbissschiene erforderlich sein, wenn beispielsweise über die Zeit neue Befunde, beispielsweise aus von der Kieferorthopädie verschiedenen medizinischen Bereichen, wie oben bereits dargelegt, hinzutreten oder sich die bestehenden Befunde aufgrund von Wechselwirkungen im Regelkreis ändern.

Zudem ist die Therapie der CMD für den Patienten im Allgemeinen wesentlich verträglicher wenn die Annäherung an die jeweilige(n) therapeutische(n) Lage(n) der Kiefer und der Kiefergelenke der jeweilige(n) Bissposition(en) (Therapieziel(e)) schrittweise, d.h. über die Erstellung einer oder mehrerer, beispielsweise über 1 bis 10, sogenannter Zwischenschienen erfolgt.

Aus diesem Grund werden in eine bevorzugte Ausführungsform des erfindungsgemäßen Verfahrens die Schritte e) bis g) einmal oder mehrmals wiederholt, bis eine oder mehrere finale Aufbissschienen erhalten werden, mit der/denen das Therapieziel im Wesentlichen erreicht wird/werden oder bis die patientenspezifischen Beschwerden weitgehend eliminiert sind. Falls aus therapeutischer Sicht auf eine Bissposition zurückgegriffen werden muss, für die noch keine (initiale) Aufbissschiene erzeugt wurde, kann diese als neue Bissposition in Schritt g) zur Erzeugung einer entsprechenden Folge-Aufbissschiene mit einbezogen werden.

Bevorzugt werden die Schritte e) bis g) des erfindungsgemäßen Verfahrens 1- bis 10-mal wiederholt.

Ein weiter Gegenstand der vorliegenden Erfindung betrifft Aufbissschienen und Folge-Aufbissschienen, zur Verwendung in der Behandlung eines Patienten mit wenigstens einer Erkrankung, die von einer funktionellen Störung des Kauorgans beruht, insbesondere Kraniomandibulärer Dysfunktionen, die nach dem hier beschriebene Verfahren hergestellt werden.

Wie bereits erwähnt eignen sich diese Aufbissschienen und Folge-Aufbissschienen in besonders vorteilhafter Weise zur Behandlung von Patienten, die unter einer Erkrankung, die von einer funktionellen Störung des Kauorgans ausgeht, wie Bruxismus und Kraniomandibulären Dysfunktionen leiden. Insbesondere eignen sich die über das erfindungsgemäße Verfahren erhältliche Aufbissschienen und Folge-Aufbissschienen zur Behandlung von Kraniomandibulären Dysfunktionen.

Die nachfolgende Figur dient der weiteren Erläuterung der Erfindung.
Figur 1:

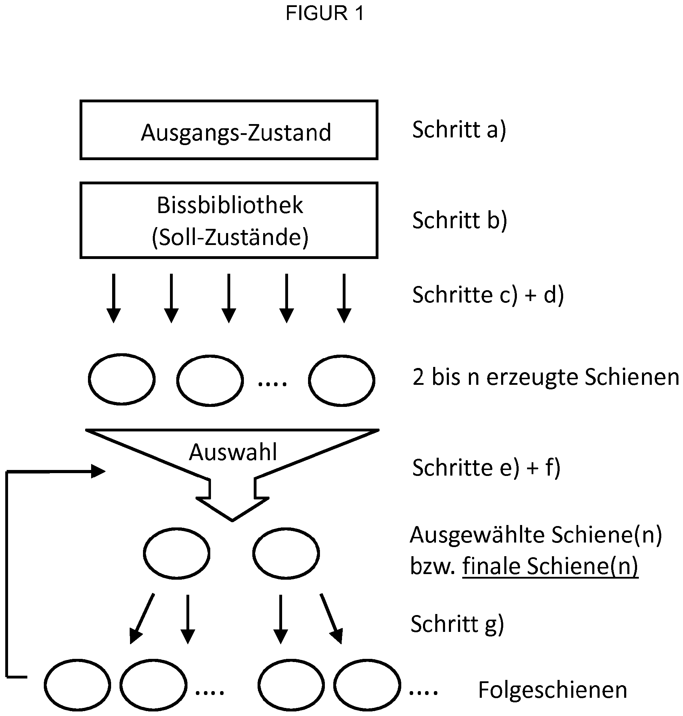

   Figur 1 zeigt eine mögliche bevorzugte Ausführungsform des erfindungsgemäßen Verfahrens. Hierbei werden in einem ersten Schritt a) zunächst Daten zur Lage der Kiefer, der Kiefergelenke, der Bewegungsbahnen des Unterkiefers und der aktuellen Morphologie der Zähne des Patienten erfasst (Ausgangs-Zustand). Anschließend werden in einem Schritt b) zwei oder mehr als zwei Bisspositionen des Patienten, d.h. für die Therapie der Kraniomandibulären Dysfunktionen relevante bzw. potentiell/vermutlich relevante Lagen des Ober- und Unterkiefers relativ zueinander, bestimmt (Soll-Zustände bzw. Bissbibliothek). Anschließend wird aus zwei oder mehr als zwei der in Schritt b) bestimmten Bisspositionen gemäß den Schritten c) und d) jeweils eine Aufbissschiene erzeugt (2 bis n erzeugte Aufbissschienen). Diese Aufbissschienen werden durch den Patienten getragen (Schritt e)). Anschließend werden in einem nachfolgenden Schritt f) eine oder mehrere dieser Aufbissschienen ausgewählt. Diese ausgewählte(n) Aufbissschiene(n) stellt/stellen entweder die finale(n) Aufbissschiene(n) und/oder, falls mit dieser/diesen nicht das gewünschte Therapieziel oder nicht alle gewünschten Therapieziele erreicht wurde(n), (eine) Initialschiene(n) dar, aus der/denen in einem nachfolgenden Schritt g) Folge-Aufbissschienen erzeugt werden. Diese Folge-Aufbissschienen werden wiederum einem Auswahlverfahren durch den Patienten (Schritte e) und f)) unterworfen. Die Schritte e), f) und, falls notwendig, Schritt g) können solange widerholt werden, bis (eine) finale Aufbissschiene(n) erhalten wird/werden mit der das/die gewünschte(n) Therapieziel(e) erreicht wird/werden.

## Patentansprüche

1. Verfahren zur Herstellung von Aufbissschienen und Folge-Aufbissschienen zur Verwendung in der Behandlung eines Patienten mit wenigstens einer Erkrankung, die von einer funktionellen Störung des Kauorgans ausgeht, insbesondere Kraniomandibulärer Dysfunktionen, enthaltend die folgenden Schritte:
a) Erfassen von Daten zur Lage der Kiefer, der Kiefergelenke, der Bewegungsbahnen des Unterkiefers und der aktuellen Morphologie der Zähne des Patienten (Ausgangs-Zustand),
b) Bestimmung von zwei oder mehr als zwei verschiedenen Bisspositionen des Patienten,
c) computergestützte Zusammenführung der in Schritt a) erfassten Daten (Ausgangs-Zustand) und der in Schritt b) bestimmten Bisspositionen (Soll-Zustände T₀) zur Berechnung jeweils eines temporären Modells einer Aufbissschiene für zwei oder mehr als zwei der in Schritt b) bestimmten Bisspositionen,
d) Erzeugung jeweils einer Aufbissschiene für zwei oder mehr als zwei der in Schritt b) bestimmten Bisspositionen, basierend auf den in Schritt c) berechneten temporären Modellen,
e) Tragen der Aufbissschienen durch den Patienten,
f) Auswahl einer oder mehrerer der in Schritt e) getragenen Aufbissschienen oder Verwerfung aller in Schritt e) getragenen Aufbissschienen und
g) Erzeugung von Folge-Aufbissschienen.

2. Verfahren nach Anspruch 1, wobei die Schritte e) bis g) einmal oder mehrmals wiederholt werden bis eine oder mehrere finale Aufbissschienen erhalten werden, mit der/denen das Therapieziel im Wesentlichen erreicht wird/werden oder bis die patientenspezifischen Beschwerden weitgehend eliminiert sind.

3. Verfahren nach einem der Ansprüche 1 oder 2, wobei die Bestimmung der Bisspositionen in Schritt b) bei unterschiedlichen Kopf- und Körperhaltungen bzw. bei verschiedenen Körperpositionen in Statik und Dynamik erfolgt.

4. Verfahren nach einem der vorhergehenden Ansprüche, wobei die Bestimmung der Bisspositionen in Schritt b) unter Einbezug eines oder mehrerer Untersuchungsbefunde aus von der Kieferorthopädie verschiedenen medizinischen Bereichen erfolgt.

5. Verfahren nach Anspruch 4, wobei die Untersuchungsbefunde ausgewählt sind unter
- aus osteopathischer Sicht optimale Kopf- und Körperhaltung,
- aus orthopädischer Sicht optimale Körperhaltung,
- aus HNO Sicht optimale Kopfhaltung und Kieferstellung,
- aus augenärztlicher Sicht optimale Kopf- und Körperhaltung.

6. Verfahren nach einem der Ansprüche 1 bis 3, wobei die verschiedenen Bisspositionen in Schritt b) berechnet werden.

7. Verfahren nach einem der vorhergehenden Ansprüche, wobei in Schritt a) gegebenenfalls zusätzlich die Form einer bestehenden Aufbissschiene und der Kontaktbereich der Zähne mit der Aufbissschiene erfasst werden.

8. Verfahren nach einem der vorhergehenden Ansprüche, wobei die Auswahl einer oder mehrerer Aufbissschienen oder Verwerfung aller Aufbissschienen in Schritt f) die folgenden Schritte umfasst:
f.1) gegebenenfalls die Erfassung von Daten zur Bestimmung der aktuellen Lage der Kiefer, der Kiefergelenke sowie der Zahnmorphologie für die getragenen Aufbissschienen und/oder die Erfassung weiterer empirischer und klinischer Daten aus von der Kieferorthopädie verschiedenen medizinischen Bereichen für die getragenen Aufbissschienen (aktueller Ist-Zustand),
f.2) Auswahl einer oder mehrerer Aufbissschienen oder Verwerfung aller Aufbissschienen anhand des aus den gegebenenfalls in Schritt f.1) erfassten Daten bestimmten objektiven patientenspezifischen Nutzens und/oder anhand des subjektiven Empfindens des Patienten.

9. Verfahren nach einem der vorhergehenden Ansprüche, wobei die Erzeugung von Folge-Aufbissschienen in Schritt g) die folgenden Schritte umfasst:
Entweder, falls in Schritt f) eine oder mehrere Aufbissschiene(n) ausgewählt wurde(n) (Initialschienen),
g.1a) die Erfassung von Daten zur Bestimmung der aktuellen Lage der Kiefer, der Kiefergelenke sowie der Zahnmorphologie für die in Schritt f) ausgewählten Aufbissschienen und/oder die Erfassung weiterer empirischer und klinischer Daten aus von der Kieferorthopädie verschiedenen medizinischen Bereichen für die in Schritt f) ausgewählten Aufbissschienen (aktueller Ist-Zustand), falls dies nicht bereits in Schritt f) erfolgt ist,
g.2a) Bestimmung und/oder Berechnung neuer Bisspositionen basierend auf den in Schritt f) ausgewählten Aufbissschienen,
g.3a) computergestützte Zusammenführung der in Schritt g.1a) erfassten Daten (aktueller Ist-Zustand) und der in Schritt g.2a) bestimmten und/oder berechneten Bisspositionen zur Berechnung jeweils eines temporären Modells einer Aufbissschiene für zwei oder mehr als zwei der in Schritt g.2a) bestimmten und/oder berechneten Bisspositionen, und
g.4a) Erzeugung jeweils einer Folge-Aufbissschiene für zwei oder mehr als zwei der in Schritt g.2a) bestimmten und/oder berechneten neuen Bisspositionen, basierend auf den in Schritt g.3a) berechneten temporären Modellen,
oder, falls in Schritt f) alle Aufbissschienen verworfen wurden,
g.1b) Bestimmung von zwei oder mehr als zwei neuen Bisspositionen des Patienten,
g.2b) computergestützte Zusammenführung der in Schritt a) erfassten Daten (aktueller Ist-Zustand) und der in Schritt g.1b) bestimmten neuen Bisspositionen zur Berechnung jeweils eines temporären Modells einer Aufbissschiene für zwei oder mehr als zwei der in Schritt g.1b) bestimmten Bisspositionen und
g.3b) Erzeugung jeweils einer Folge-Aufbissschiene für zwei oder mehr als zwei der in Schritt g.1b) bestimmten Bisspositionen, basierend auf den in Schritt g.2b) berechneten temporären Modellen.

10. Verfahren nach einem der vorhergehenden Ansprüche, wobei die Aufbissschienen und Folge-Aufbissschienen einen Aufnahmebereich zum Aufnehmen von Zähnen eines ersten Kiefers des Patienten und einen Kontaktbereich zum Kontaktieren von Zähnen eines dem ersten Kiefer gegenüberliegend angeordneten zweiten Kiefers des Patienten aufweisen.

11. Verfahren nach einem der vorhergehenden Ansprüche, wobei die Schritte e) bis g) 1- bis 10-mal wiederholt werden.

12. Aufbissschienen und Folge-Aufbissschienen, zur Verwendung in der Behandlung eines Patienten mit wenigstens einer Erkrankung, die von einer funktionellen Störung des Kauorgans ausgeht, insbesondere Kraniomandibulärer Dysfunktionen, erhalten durch ein Verfahren nach einem der vorhergehenden Ansprüche.
